Europäisches Patentamt

European Patent Office

Office européen des brevets

(19)

(11) Veröffentlichungsnummer : **0 334 904 B1**

(12) # EUROPÄISCHE PATENTSCHRIFT

(45) Veröffentlichungstag der Patentschrift :
27.05.92 Patentblatt 92/22

(51) Int. Cl.$^5$ : **A61L 2/00**, // C12N15/00

(21) Anmeldenummer : **88905801.2**

(22) Anmeldetag : **04.07.88**

(86) Internationale Anmeldenummer :
**PCT/EP88/00592**

(87) Internationale Veröffentlichungsnummer :
**WO 89/03226 20.04.89 Gazette 89/09**

(54) **VERFAHREN ZUR INAKTIVIERUNG REKOMBINANTER DNA.**

(30) Priorität : **07.10.87 DE 3733921**

(43) Veröffentlichungstag der Anmeldung :
**04.10.89 Patentblatt 89/40**

(45) Bekanntmachung des Hinweises auf die
Patenterteilung :
**27.05.92 Patentblatt 92/22**

(84) Benannte Vertragsstaaten :
**AT BE CH DE FR GB IT LI LU NL SE**

(56) Entgegenhaltungen :
**EP-A- 0 189 587**
**Chemical Abstracts, volume 105, Nr. 5, 4.August 1986, (Coulumbus, Ohio, US) F. Zhang et al.: "Study of inactivation ofhepatitis B surface antigen (HBsAg) with different disinfectants", see abstract 40658p, & Zhonghua Yufangyixe Zazhi 1984,18(1), 10-12**
**Chemical Abstracts, volume 104, Nr. ,31 March 1986, (Columbus, Ohio, US), W.M. Waites: "Inactivation of spores with chemicalagents", see page 400, abstract 105993v, & FEMS Symp. 1985, 18, 383-96**

(73) Patentinhaber : **BOEHRINGER MANNHEIM GMBH**
**Sandhofer Strasse 112-132**
**W-6800 Mannheim-Waldhof (DE)**

(72) Erfinder : **POPP, Friedrich**
**Irmfridweg 2**
**W-8121 Sindelsdorf (DE)**
Erfinder : **COMER, Michael, James**
**Eichenstr. 4**
**W-8139 Bernried (DE)**
Erfinder : **SCHUMACHER, Günther**
**Kapellenweg 20**
**W-8139 Bernried (DE)**
Erfinder : **MUNSTER, Michael, John**
**Grube 17**
**W-8122 Penzberg (DE)**
Erfinder : **SEYDLER, Bodo**
**Herzogstandstr. 35**
**W-8120 Weilheim (DE)**

(74) Vertreter : **Huber, Bernhard, Dipl.-Chem. et al**
**Patentanwälte H. Weickmann, Dr. K. Fincke F.A. Weickmann, B. Huber Dr. H. Liska, Dr. J. Prechtel Kopernikusstrasse 9 Postfach86 08 20**
**W-8000 München 86 (DE)**

## Beschreibung

Die Erfindung betrifft ein Verfahren zur Inaktivierung der biologischen Aktivität von DNA, insbesondere rekombinanter DNA.

Gemäß den Richtlinien zum Schutz vor Gefahren durch in vitro neu kombinierte Nucleinsäuren, die vom Bundesminister für Forschung und Technologie herausgegeben werden, sind Abfälle, die Nucleinsäuren, also DNA enthalten, zu sterilisieren bzw. zu denaturieren. Deshalb ist es beim Arbeiten mit rekombinante DNA-enthaltenden Mikroorganismen nicht nur erforderlich, die Organismen zu inaktivieren, sondern auch die gegebenenfalls rekombinante DNA in den Organismen zu zerstören. Die Abtötung von Mikroorganismen gelingt in der Regel relativ einfach. Durch die angewandten Maßnahmen wird jedoch die DNA in den Mikroorganismen in der Regel nicht zerstört. Da nun diese Sterilisation bzw. Denaturierung erfolgen muß, bevor die Biomasse in die Kläranlage eingeleitet wird, sind außerdem Maßnahmen zu treffen, damit keine Substanzen mit der Biomasse in die nachgeschaltete Kläranlage gelangen, die beispielsweise die Mikroorganismen des Belebtschlammes schädigen oder abtöten, falls eine biologische Kläranlage nach dem Belebtschlammverfahren beschickt wird, was bevorzugt ist. Es muß also Sorge getragen werden, daß die verwendeten Substanzen nicht toxisch sind oder vor Einleitung in die Kläranlage so abgebaut werden, daß sie keinen Schaden verursachen.

Die bisher bekannten und z.B. vom Bundesgesundheitsamt (BGA) der Bundesrepublik Deutschland und von der Zentralkommission für Biologische Sicherheit (ZkBS) der Bundesrepublik Deutschland anerkannten Methoden zur Sterilisation und Inaktivierung (z.B. Dampfsterilisation über 20 min bei 121°C) sind sehr aufwendig. Insbesondere bei der Behandlung von industriell üblichen Fermentationsmengen von 10 bis 50 m³ sind diese Verfahren sowohl apparativ als auch wegen des hohen Energieverbrauchs sehr kostenintensiv.

Aufgabe der vorliegenden Erfindung war es daher, ein Verfahren zur Verfügung zu stellen, mit dem die biologische Aktivität von DNA, insbesondere rekombinanter DNA, sicher inaktiviert werden kann, ohne daß unerwünschte, insbesondere toxische Substanzen in das Abwasser gelangen. Außerdem soll das Verfahren kostengünstig durchführbar sein.

Diese Aufgabe wird gelöst durch ein Verfahren zur Inaktivierung der biologischen Aktivität von DNA, insbesondere rekombinanter DNA, das dadurch gekennzeichnet ist, daß man der DNA enthaltenden Biomasse eine Percarbonsäure mit 1 bis 3 Kohlenstoffatomen, ein Alkaliperoxid oder ein Alkaliperoxomonosulfat zugibt und die Mischung dann auf 60 bis 100°C erhitzt.

Überraschenderweise wurde festgestellt, daß durch Anwendung der obengenannten Verbindungen und durch Erhitzung des Ansatzes nicht nur noch lebende Mikroorganismen abgetötet werden, sondern daß auch die in ihnen enthaltene DNA so weit abgebaut wird, daß keine replikationsfähigen Bruchstücke mehr erhalten bleiben.

Mit dem erfindungsgemäßen Verfahren kann ein Abbau der DNA auf Bruchstücke kleiner/gleich 500 bp erreicht werden, was auch von den von der ZKBS zugelassenen Entsorgungsverfahren (Autoklavieren) nicht übertroffen wird. Derartige Bruchstücke sind nicht mehr biologisch aktiv und können daher gefahrlos in das Abwasser geleitet werden.

Das erfindungsgemäße Verfahren kann vorteilhaft angewendet werden für Biomassen, wie sie bei der Züchtung und Herstellung von rekombinanter DNA innerhalb oder außerhalb von tierischen oder menschlichen Zellen, sowie von Mikroorganismen entstehen. Abwässer, die Mikroorganismen, Plasmide und dergleichen DNA-haltiges Material enthalten, können daher vor der Einführung in die eigentliche Kläranlage der erfindungsgemäßen Vorbehandlung unterzogen werden, die sicherstellt, daß keine DNA in replikationsfähiger Form mehr vorhanden sein kann.

Die Biomasse, wie beispielsweise eine Mikroorganismen enthaltende Kulturlösung, wird mit einer Percarbonsäure mit 1 bis 3 Kohlenstoffatomen, einem Alkaliperoxid oder einem Alkaliperoxomonosulfat versetzt. Bevorzugt wird dabei Peressigsäure angewendet, da diese einerseits relativ stabil ist und andererseits kostengünstig ist. Ebenso sind die Perameisensäure und Perpropionsäure geeignet. Als Alkali wird im Rahmen der Erfindung Na, K und Li bevorzugt. Auch die Ammoniumsalze werden hier zu den Alkalisalzen gezählt. Bevorzugt wird die Perverbindung in einer Konzentration von 0,05 bis 5 Gew.-% eingesetzt. Die Konzentration hängt dabei von der verwendeten Verbindung ab sowie von der zu behandelnden Biomasse.

Nach Zugabe der Perverbindung wird die Mischung erhitzt. Eine Inaktivierung wird erreicht durch Erhitzen auf eine Temperatur zwischen 60 und 100°C. Bevorzugt wird auf eine Temperatur zwischen 70 und 90°C erhitzt. Insbesondere bei Verwendung von Peressigsäure ist es vorteilhaft, die Mischung auf mindestens 70°C zu erhitzen, da bei dieser Temperatur die Peressigsäure rasch vollständig zersetzt wird.

Die Erhitzung der Mischung erfolgt bevorzugt so lange, bis chromatographisch keine DNA mit einer Kettenlänge von mehr als 500 Basenpaaren mehr nachweisbar ist. Dies ist in der Regel nach 20 bis 60 Minuten der Fall. Um zu überprüfen, ob alle DNA abgebaut wurde, wird aus der Mischung ein Aliquot entnommen und damit eine Agarosegel-Elektrophorese durchgeführt. Die chromatographische Auftrennung nach Molekulargewicht zeigt dann an, welche Bruchstücke in der Lösung vorhanden sind.

Das erfindungsgemäße Verfahren wird vorteilhaft im pH-Bereich zwischen 6 und 11, besonders bevorzugt zwischen pH 7 und 10 durchgeführt.

Ein wesentlicher Vorteil des erfindungsgemäßen Verfahrens ist, daß die verwendeten Perverbindungen vollständig abgebaut werden. Aus den verwendeten Verbindungen entstehen nur biologisch harmlose und untoxische Verbindungen, die sogar direkt in die Kanalisation geleitet werden können und bei denen außerdem keine Gefahr besteht, daß die im Belebtschlamm enthaltenen Mikroorganismen abgetötet werden.

Die Erfindung wird noch durch die folgenden Beispiele in Verbindung mit der beigefügten Zeichnungen von Elektropherogrammen erläutert.

Fig. 1 zeigt ein Elektropherogramm von verschiedenen Proben, bei denen verschiedene Perverbindungen angewendet wurden (Tabelle 1).

Fig. 2 zeigt ein Elektorpherogramm verschiedener Proben, bei denen verschiedene Perverbindungen angewendet wurden (Tabelle 2).

Fig. 3 zeigt ein Elektropherogramm von Proben, bei denen verschiedene Perverbindungen mit höherer Konzentration und geänderter Inkubationszeit angewendet wurden (Tabelle 3).

Fig. 4 zeigt ein Elektropherogramm von Proben, bei denen verschiedene Peressigsäure-Konzentrationen angewendet wurden (Tabelle 4).

Fig. 5 zeigt ein Elektropherogramm von Proben, bei denen verschiedene Konzentrationen von Peroxomonosulfat angewendet wurden (Tabelle 5).

Fig. 6 zeigt ein Elektropherogramm von Proben, bei denen Peressigsäure bei verschiedenen Temperaturen angewendet wurde (Tabelle 6).

Fig. 7 zeigt ein Elektropherogramm von Proben, bei denen Peressigsäure verschiedener Konzentration angewendet wurde (Tabelle 7).

Fig. 8 zeigt ein Elektropherogramm von Proben von DNA einer eukaryotischen Zellinie, die mit Peressigsäure behandelt wurde (Tabelle 8 und 9).

## Beispiel 1

Es wurde die Zersetzung von Plasmid DNA durch Perverbindungen getestet. Dazu wurde das Plasmid pBR322 (PNAS, USA 75 (1978) 373) verwendet. Von diesem Plasmid wurden Lösungen eingesetzt, die 30 µg Plasmid DNA/ml enthielten. Diese Proben wurden in verschiedenen Ansätzen mit Perverbindungen behandelt. 100 µl des entsprechenden Ansatzes wurden unter den in den folgenden Tabellen jeweils angegebenen Bedingungen inkubiert. Anschließend wurden jeweils 20 µl des Ansatzes entnommen, mit 5 µl Probenpuffer vermischt und auf eine Agarosegel-Elektrophoreseplatte (0,8 Gew.-% Agarose; 0,04 Mol/l TRIS/Acetatpuffer, pH 8; 0,001 Mol/l EDTA) aufgegeben und, wie in Maniatis T., Fritsch E.S. und Sambrook J. in Molecular Cloning, Cold Spring Harbor Laboratory, New York 11724 (1982), Seite 454 angegeben, aufgetrennt. Als Vergleichslösungen wurden 0,6 µg pBR322 in 20 µl TE-Puffer und 5 µl Probenpuffer verwendet. Als Längenstandard (λ-Längenstandard) wurde Phage Lamdba (J.Mol.Biol. 162 (1982) 729-773), geschnitten mit Hind III (erhältlich als DNA-Längenstandard II, Bestell-Nr. 236 250, Boehringer Mannheim GmbH) eingesetzt. Die Molekulargewichte der Fragmente sind: 15.27, 6,21, 4.41, 2.88, 1.53, 1.34, 0.37 Megadalton entsprechend 23130, 9416, 6682, 4361, 2322, 2027, 564 Basenpaare. Die verwendeten Perverbindungen, Konzentrationen und Inkubationsbedingungen sind den folgenden Tabellen 1 bis 5 zu entnehmen:

TABELLE 1

| Spur | Perverbindung | Temperatur/ Inkubationszeit |
|------|---------------|------------------------------|
| 1 | --- | Raumtemperatur (RT)/60 min |
| 2 | Na-peroxid, 0,1 % | " |
| 3 | Harnstoffperoxid, 0,1 % | " |
| 4 | Na-perborat, 0,1 % | " |
| 5 | Peressigsäure, 0,1 % | " |
| 6 | $\lambda$-Längenstandard | --- |
| 7 | --- | 80°C/30 min |
| 8 | Na-Peroxid, 0,1 % | " |
| 9 | Harnstoffperoxid, 0,1 % | " |
| 10 | Na-Perborat, 0,1 % | " |
| 11 | Peressigsäure, 0,1 % | " |

TABELLE 2

| Spur | Perverbindung | Temperatur/ Inkubationszeit |
|------|---------------|------------------------------|
| 1 | --- | Raumtemperatur (RT)/60 min |
| 2 | Peressigsäure, 0,1 % | " |
| 3 | Perform*, 0,1 % | " |
| 4 | $H_2O_2$, 0,1 % | " |
| 5 | $\lambda$-Längenstandard | --- |
| 6 | --- | 80°C/30 min |
| 7 | Peressigsäure, 0,1 % | " |
| 8 | Perform*, 0,1 % | " |
| 9 | $H_2O_2$, 0,1 % | " |

* = Peroxomonosulfat

4

TABELLE 3

| Spur | Perverbindung | Temperatur/<br>Inkubationszeit |
|------|---------------|-------------------------------|
| 1 | --- | RT/60 Minuten |
| 2 | Peressigsäure, 0,5 % | " |
| 3 | Perform, 0,5 % | " |
| 4 | $H_2O_2$, 0,5 % | " |
| 5 | $\lambda$ -Längenstandard | " |
| 6 | --- | --- |
| 7 | Peressigsäure, 0,5 % | 80°C/30 Minuten |
| 8 | Perform, 0,5 % | " |
| 9 | $H_2O_2$, 0,5 % | " |

TABELLE 4

| Spur | Konzentration Peressigsäure (Gew.-%) |
|------|--------------------------------------|
| 1 | 0 % (Raumtemperatur) |
| 2 | 0 % |
| 3 | 0,9 % |
| 4 | 0,5 % |
| 5 | 0,1 % |
| 6 | 0,05 % |
| 7 | 0,025% |
| 8 | $\lambda$ -Längenstandard |

TABELLE 5

Spur    Konzentration Perform (Peroxomonosulfat) (Gew.-%)

| Spur | Konzentration |
|---|---|
| 1 | 0 % (Raumtemperatur) |
| 2 | 0 % |
| 3 | 0,9 % |
| 4 | 0,5 % |
| 5 | 0,1 % |
| 6 | 0,05 % |
| 7 | $\lambda$ -Längenstandard |

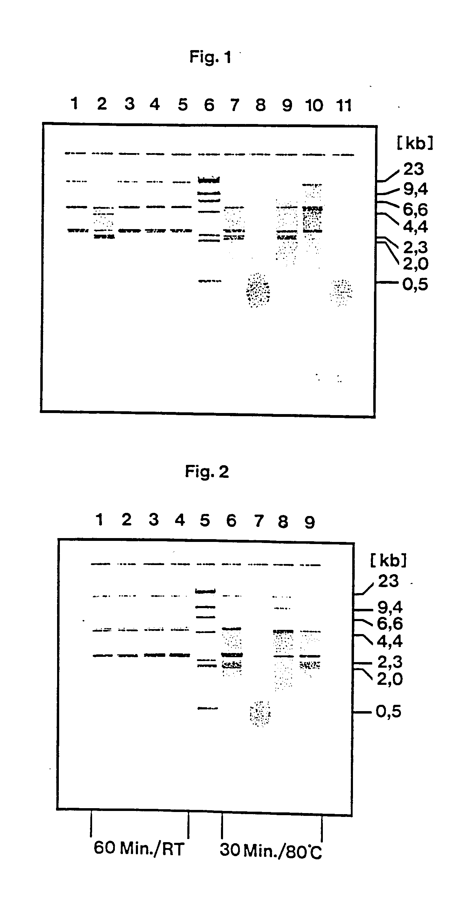

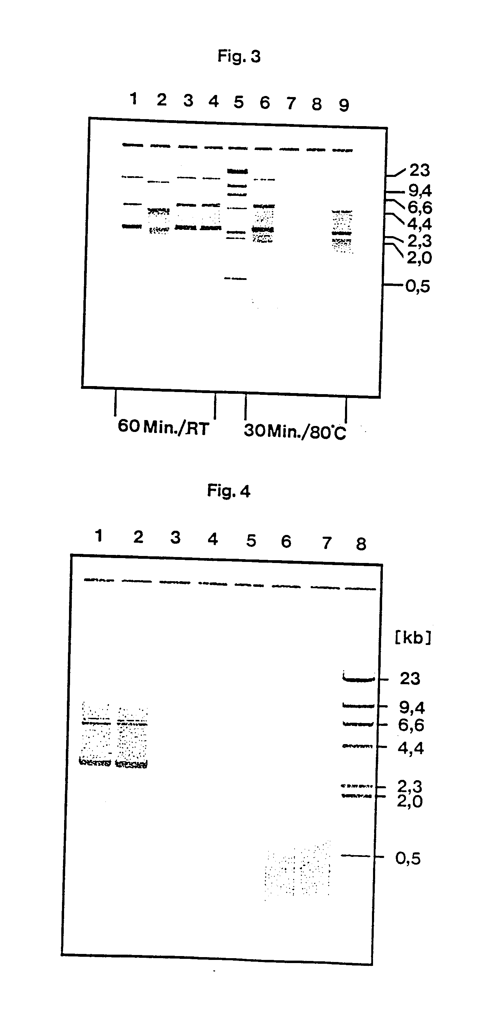

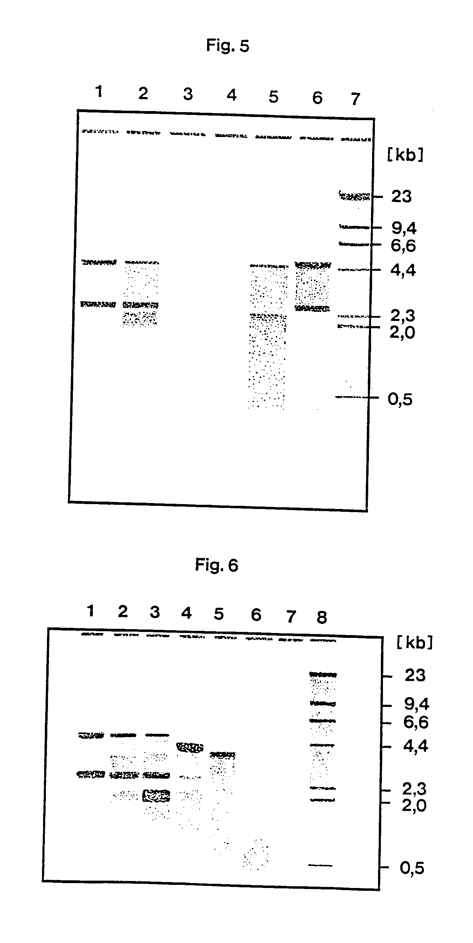

Die Ergebnisse sind den Figuren 1 bis 5 zu entnehmen. Aus Figur 1 und 2 ergibt sich dabei, daß bei einer Konzentration von 0,1 % Perverbindung, bei einer Inkubationszeit von 30 Minuten und bei einer Temperatur von 80°C nur bei Verwendung von Peressigsäure und Natriumperoxid als Perverbindung keine DNA-Fragmente mit einer Länge über 500 Basenpaare (bp) nachweisbar sind. Figur 3 zeigt, daß die Verwendung von Peroxomonosulfat einer Konzentration von 0,5 % eine befriedigende Fragmentierung der Plasmid-DNA ergibt. Aus Figur 4 ist zu entnehmen, daß die Anwendung von Peressigsäure mit einer Konzentration von 0,1 % bei einer Temperatur von 80°C und einer Inkubationszeit von 30 Minuten zu einer ausreichenden Zersetzung der Plasmid-DNA führt, so daß keine Fragmente mit einer Länge von mehr als 500 Basenpaare im Gelbild erkennbar sind. Figur 5 zeigt, daß bei Anwendung von Peroxomonosulfat mit einer Konzentration von 0,5 % bei einer Temperatur von 80°C und einer Inkubationszeit von 30 Minuten ebenfalls eine ausreichende Fragmentierung der Plasmid-DNA erreicht wird.

**Beispiel 2**

Lösungen von Plasmid pBR322 wurden, wie in Beispiel 1 beschrieben, mit 0,05 % Peressigsäure 30 Minuten lang bei unterschiedlichen Temperaturen inkubiert. Abweichend von Beispiel 1 wurden jedoch statt 30 μg Plasmid-DNA/ml 50 μg Plasmid-DNA/ml eingesetzt. Die jeweils angewendeten Temperaturen sind der folgenden Tabelle 6 zu entnehmen:

TABELLE 6

Spur    Temperatur

| Spur | Temperatur |
|---|---|
| 1 | RT, ohne Peressigsäure |
| 2 | 80°C, ohne Peressigsäure |
| 3 | RT |
| 4 | 50°C |
| 5 | 60°C |
| 6 | 70°C |
| 7 | 80°C |
| 8 | $\lambda$ -Längenstandard |

Die Lösungen wurden dann, wie in Beispiel 1 beschrieben, auf einer Agarosegel-Elektrophoreseplatte aufgetrennt. Als Vergleichslösungen wurden 1 µg pBR322 in 20 µl TE-Puffer und 5 µl Probenpuffer verwendet. Die Konzentration des Längenstandards war analog zu Beispiel 1. Die Ergebnisse sind Figur 6 zu entnehmen. Dabei zeigt sich, daß bei einer Inkubationszeit von 30 Minuten bereits bei 60°C eine weitgehende Fragmentierung der Plasmid-DNA erreicht wird und daß bei 70°C im Gelbild keine Fragmente mehr erkennbar sind, die größer als 500 bp sind.

**Beispiel 3**

Der transformierte Mikroorganismus E.coli ED/pBT2a-I (DSM 3143) wurde, wie in EP 187138 beschrieben, fermentiert. 0,1 ml Kulturbrühe wurden entnommen, erfindungsgemäß bei 80°C 30 Minuten lang in verschiedenen Konzentrationen von Peressigsäure inkubiert und anschließend mit 10 µl einer 10 mg/ml Proteinase K enthaltenden Lösung versetzt und bei 37°C 30 Minuten lang zur Ablösung membrangebundener DNA inkubiert. Nach Zentrifugieren (Eppendorf Zentrifuge 14000 Upm/5 min) wurden je 20 µl dieser so behandelten Kulturbrühe zur Elektrophorese eingesetzt. Die Konzentrationen der Peressigsäure, sowie die jeweils verwendeten Inkubationsbedingungen sind der folgenden Tabelle 7 zu entnehmen:

```
TABELLE 7:
```

| Spur | Konzentration Peressigsäure (Gew.-%) | Temperatur/ Inkubationszeit |
|------|--------------------------------------|------------------------------|
| 1 | zentrifugierte Kulturbrühe vor Inkubation | |
| 2 | 0 % | 80°C/30 Min. |
| 3 | 0,1 % | RT/ 2 Std. |
| 4 | 0,1 % | 80°C/30 Min. |
| 5 | 0,2 % | " |
| 6 | 0,5 % | " |
| 7 | 1 % | " |
| 8 | λ-Längenstandard | --- |

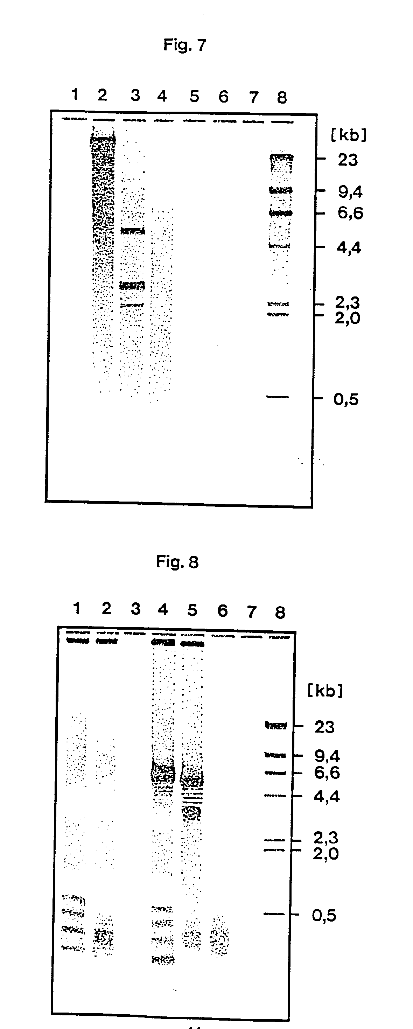

Die Ergebnisse sind in Figur 7 gezeigt. Es zeigt sich, daß auch in einer Kulturbrühe nach Behandlung mit 0,2 % Peressigsäure bei einer Inkubationszeit von 30 Minuten und einer Temperatur von 80°C keine DNA-Fragmente mit mehr als 500 bp nachweisbar sind.

**Beispiel 4**

a) Inaktivierung von eukaryotischen Zellen mit Peressigsäure

Die Hybridomzellen ECACC 84122003 wurden, wie in EP-A-150309 beschrieben, gezüchtet. In verschiedenen Ansätzen wurden Lösungen dieser Hybridomzellen mit Peressigsäure inkubiert. Die Bedingungen sind der folgenden Tabelle 8 zu entnehmen:

TABELLE 8

| Spur | Konzentration Peressigsäure | Temperatur/Inkubaticnszeit |
|---|---|---|
| 1 | 0 % | Raumtemperatur |
| 2 | 0 % | 80°C/30 Min. |
| 3 | 0,2 % | 80°C/30 Min. |

Als Proben 1 bis 3 wurden je 20 µl einer Suspension von $10^7$ Zellen/100 µl auf eine Agarosegel-Elektrophoreseplatte aufgetragen und aufgetrennt. Das Ergebnis ist in Figur 8 dargestellt. Es zeigt sich, daß die Behandlung mit Peressigsäure einer Konzentration von 0,2 % bei einer Inkubationstemperatur von 80°C und einer Inkubationsdauer von 30 Minuten die DNA so zerstört, daß keine DNA-Fragmente mit einer Länge über 500 bp mehr nachweisbar sind.

b) Überprüfung der Transformationsfähigkeit von pBR322 in Gegenwart von eukaryotischen Zellen nach Behandlung mit Peressigsäure

Als Probelösungen wurden Suspensionen von $10^7$ der gemäß a) erhaltenen Hybridomzellen pro 0,1 ml, die zusätzlich 5 µg pBR322/0,1 ml enthielten, verwendet. Diese Lösungen wurden mit Peressigsäure behandelt. Die Inkubationsbedingungen sind der folgenden Tabelle 9 zu entnehmen:

TABELLE 9

| Spur | Konzentration Peressigsäure | Temperatur/Inkubatkionszeit |
|---|---|---|
| 4 | 0 % | Raumtemperatur |
| 5 | 0 % | 80°C/30 Min |
| 6 | 0,2 % | " |
| 7 | 1 % | " |
| 8 | $\lambda$ -Längenstandard | --- |

Je 20 µl dieser Mischungen wurden durch Agarosegel-Elektrophorese aufgetrennt. Die Ergebnisse sind der Figur 8 zu entnehmen. Es zeigt sich, daß Peressigsäure einer Konzentration von 0,2 % bei einer Inkubationszeit von 30 Minuten und einer Inkubationstemperatur von 80°C zu einer befriedigenden Zerstörung der DNA führt, so daß keine DNA-Fragmente mit mehr als 500 bp mehr enthalten sind.

Von den gemäß b) hergestellten Proben 4 bis 7 wurde nach der Inkubation mit Peressigsäure jeweils ein Teil gegen TE-Puffer, pH 7,5, dialysiert. Von den hierbei erhaltenen Lösungen wurden je 20 µl zu einer Transformation von E.coli HB101 (DSM 1607) über die bei Maniatis "Molecular Cloning" Seiten 250-251 beschriebene Calciumchloridmethode transformiert.

Die verwendeten Bedingungen, sowie die erhaltenen Lebendkeimzahlen (LKZ) sind der folgenden Tabelle 10 zu entnehmen:

TABELLE 10:

| Probe | Zellart | Inkubationsbedingungen | | | LKZ |
|---|---|---|---|---|---|
| | | Peressigsäure | Temperatur | Zeit | |
| 1 | pBR322[a)] | 0 | -- | -- | $1,4 \times 10^6$ |
| 2 | Zellkul-turlösung[b)] | 0 | RT | -- | $1 \times 10^3$ |
| 3 | " | 0 | 80°C | 30 min | $5 \times 10^5$ |
| 4 | " | 0,2 % | 80°C | 30 min | 0 |
| 5 | " | 1 % | 80°C | 30 min | 0 |

a) reine Plasmidlösung 1 µg in 20 µl TE-Puffer, pH 7,5

b) Plasmid in Zellkulturlösung (partiell verdaut durch

Nukleasen; dialysiert; davon 20 µl in <u>E.coli</u> trans-formiert)

Zur Bestimmung der LKZ wurden die die Bakterien enthaltenden Lösungen in $10^{-1}$ bis $10^{-6}$-facher Verdünnung auf Agar-Platten mit LB-Medium mit und ohne Ampicillin (50 µg/ml) ausplattiert, über Nacht bei 37°C bebrütet und anschließend die Anzahl der gewachsenen Kolonien visuell ermittelt.

Es zeigt sich, daß die Inkubation mit 0,2 %iger Peressigsäure bei einer Inkubationstemperatur von 80°C und einer Inkubationsdauer von 30 Minuten zu einer vollständigen Abtötung der Zellen führt.

**Patentansprüche**

1. Verfahren zur Inaktivierung der biologischen Aktivität von DNA, insbesondere rekombinanter DNA, **dadurch gekennzeichnet,** daß man der DNA enthaltenden Biomasse eine Percarbonsäure mit 1 bis 3 C-Atomen, eines ihrer Salze, ein Alkaliperoxid oder ein Alkaliperoxomonosulfat zusetzt und die Mischung anschließend auf 60 bis 100°C erhitzt.

2. Verfahren nach Anspruch 1, **dadurch gekennzeichnet,** daß man die Perverbindung in einer Menge von 0,05 bis 5 % verwendet.

3. Verfahren nach Anspruch 1 oder 2, **dadurch gekennzeichnet,** daß die Erhitzung so lange erfolgt, bis chromatographisch keine DNA mit einer Kettenlänge von mehr als 500 Basenpaaren mehr nachweisbar ist.

4. Verfahren nach einem der Ansprüche 1 bis 3, **dadurch gekennzeichnet,** daß der pH-Wert im Bereich von 6 bis 11 liegt.

5. Verfahren nach Anspruch 4, **dadurch gekennzeichnet,** daß der pH-Wert im Bereich von 7 bis 10 liegt.

**Claims**

1. Process for the inactivation of the biological activity of DNA, especially of recombinant DNA, characterised in that one adds to the DNA-containing biomass a percarboxylic acid with 1 to 3 C-atoms, one of its salts, an alkali metal peroxide or an alkali metal peroxomonosulphate and subsequently heats the mixture to 60 to 100°C.

2. Process according to claim 1, characterised in that one uses the per compound in an amount of 0.05 to 5%.

3. Process according to claim 1 or 2, characterised in that the heating takes place until no more DNA with a chain length of more than 500 base pairs is detectable chromatographically.

4. Process according to one of claims 1 to 3, characterised in that the pH value lies in the range of 6 to 11.

5. Process according to claim 4, characterised in that the pH value lies in the range of 7 to 10.


**Revendications**

1. Procédé d'inactivation de l'activité biologique de l'ADN, en particulier de l'ADN recombinant, caractérisé en ce que l'on ajoute à la biomasse contenant de l'ADN un peracide carboxylique de 1 à 3 atomes de carbone, l'un de ses sels, un peroxyde alcalin ou un peroxomonosulfate alcalin et l'on chauffe ensuite le mélange entre 60 et 100°C.

2. Procédé selon la revendication 1, caractérisé en ce que l'on utilise le percomposé en une quantité de 0,05 à 5%.

3. procédé selon la revendication 1 ou 2, caractérisé en ce que le chauffage a lieu jusqu'à ce qu'aucun ADN d'une longueur de chaîne supérieure à 500 paires de bases ne puisse plus être mis en évidence par chromatographie.

4. Procédé selon l'une des revendications 1 à 3, caractérisé en ce que le pH est situé dans le domaine de 6 à 11.

5. Procédé selon la revendication 4, caractérisé en ce que le pH est situé dans le domaine de 7 à 10.

Fig. 1

Fig. 2

60 Min./RT          30 Min./80°C

## Fig. 3

1  2  3  4  5  6  7  8  9

23
9,4
6,6
4,4
2,3
2,0

0,5

60 Min./RT    30 Min./80°C

## Fig. 4

1   2   3   4   5   6   7   8

[kb]

23

9,4
6,6

4,4

2,3
2,0

0,5

## Fig. 5

## Fig. 6

## Fig. 7

## Fig. 8